(19) Europäisches Patentamt
European Patent Office
Office européen des brevets

(11) Numéro de publication: **0 423 032 A2**

(12) # DEMANDE DE BREVET EUROPEEN

(21) Numéro de dépôt: **90402841.2**

(22) Date de dépôt: **11.10.90**

(51) Int. Cl.5: **C07D 487/04**, A61K 31/55, //(C07D487/04,243:00,235:00)

(30) Priorité: **12.10.89 GB 8923008**

(43) Date de publication de la demande: **17.04.91 Bulletin 91/16**

(84) Etats contractants désignés: **AT BE CH DE DK ES FR GB GR IT LI LU NL SE**

(71) Demandeur: **ROUSSEL-UCLAF**
**35, boulevard des Invalides**
**F-75007 Paris(FR)**

(72) Inventeur: **Hedgecock, Charles John Robert**
**186 High Street**
**Wootton Basset, Wiltshire SN4 7BZ(GB)**
Inventeur: **Kuo, Elizabeth Anne**
**8 Bullfinch Close**
**Dorcan, Wiltshire(GB)**

(74) Mandataire: **Vieillefosse, Jean-Claude et al**
**Roussel-Uclaf 111, route de Noisy B.P. 9**
**F-93230 Romainville(FR)**

(54) **Nouveau procédé de préparation d'isoxazolyl et d'isothiazolyl imidazo-benzodiazépines, nouveaux dérivés obtenus, application à titre de médicament de ces nouveaux produits et les compositions les renfermant.**

(57) L'invention concerne un procédé pour préparer

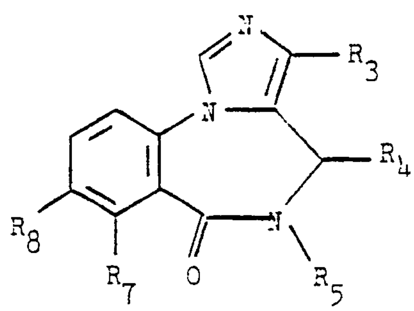

(I)

dans laquelle $R_3$ représente

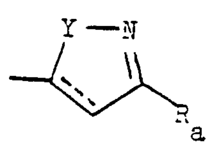

dans lequel $R_a$ représente un hydrogène, un alkyle ($C_{1-5}$), cycloalkyle ($C_{3-6}$) ou phényle, Y représente un oxygène ou un soufre, le pointillé représente une liaison éventuelle, $R_4$ et $R_5$ représentent un hydrogène, un alkyle ($C_{1-5}$), un alkényle ($C_{2-5}$) ou forment ensemble un alkylène ou alkénylène ($C_{2-5}$), $R_7$ et $R_8$ représentent un hydrogène, un halogène, un radical nitro ou nitrile ou $CX_3$ dans lequel X représente un halogène ainsi que leurs sels avec les acides.

Les produits (I) possèdent d'intéressantes propriétés pharmacologiques et sont utiles comme médicaments notamment dans le traitement des troubles de la mémoire.



Xerox Copy Centre

# NOUVEAU PROCÉDÉ DE PRÉPARATION D'ISOXAZOLYL ET D'ISOTHIAZOLYL IMIDAZOBENZODIAZÉPINES, NOUVEAUX DÉRIVÉS OBTENUS, APPLICATION À TITRE DE MÉDICAMENT DE CES NOUVEAUX PRODUITS ET LES COMPOSITIONS LES RENFERMANT.

La présente invention concerne un nouveau procédé de préparation d'imidazobenzodiazépines, les nouveaux dérivés obtenus, l'application à titre de médicaments de ces nouveaux produits et les compositions les renfermant.

Le brevet américain USP 4 775 671 décrit des imidazobenzodiazépines substituées en position 3 par des groupements hétérocycliques. La demanderesse à trouvé un nouveau procédé de préparation de ces produits qui permet de cycliser directement le groupement hétérocyclique avec de bons rendements.

L'invention a ainsi pour objet un nouveau procédé de préparation des produits de formule générale (I) :

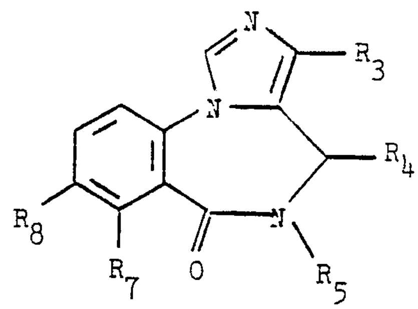

(I)

dans laquelle $R_3$ représente un groupement

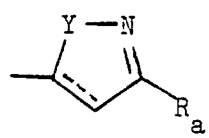

dans lequel $R_a$ représente un atome d'hydrogène, un groupement alkyle, linéaire ou ramifié renfermant de 1 à 5 atomes de carbone, un groupement cycloalkyle renfermant de 3 à 6 atomes de carbone, un groupement phényl, Y représente un atome d'oxygène ou de soufre, le trait pointillé représente une seconde liaison éventuelle,

$R_4$ et $R_5$, identiques ou différents, représentent un atome d'hydrogène, un radical alkyle linéaire ou ramifié renfermant de 1 à 5 atomes de carbone, un radical alkényl linéaire ou ramifié renfermant de 2 à 5 atomes de carbone, ou $R_4$ et $R_5$ forment ensemble un groupement alkylène ou alkénylène renfermant de 2 à 5 atomes de carbone,

$R_7$ et $R_8$, identiques ou différents, représentent un atome d'hydrogène, un atome d'halogène ou un groupement $CX_3$ dans lequel X représente un atome d'halogène ou encore un groupement nitro ou nitrile ainsi que de leurs sels d'addition avec les acides minéraux ou organiques,

procédé de préparation caractérisé en ce que l'on fait réagir un produit de formule (II) :

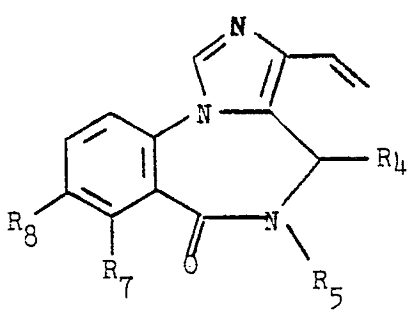

(II)

dans laquelle $R_4$, $R_5$, $R_7$ et $R_8$ ont la signification déjà indiquée, avec un produit de formule (III) :

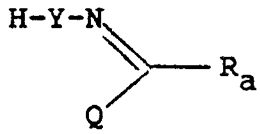

(III)

dans laquelle Y et $R_a$ ont la signification déjà indiquée et Q représente un groupement éliminable, pour obtenir un produit de formule ($I_A$) :

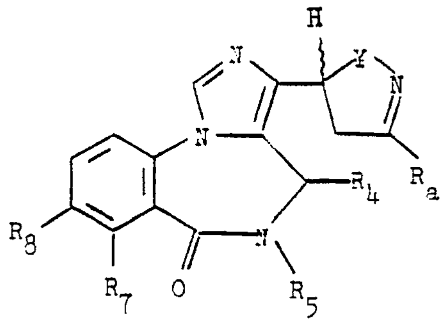

($I_A$)

dans laquelle Y, $R_a$, $R_4$, $R_5$, $R_7$ et $R_8$ ont la signification déjà indiquée, puis le cas échéant, fait réagir ce dernier avec un oxydant doux pour obtenir un produit de formule ($I_B$) :

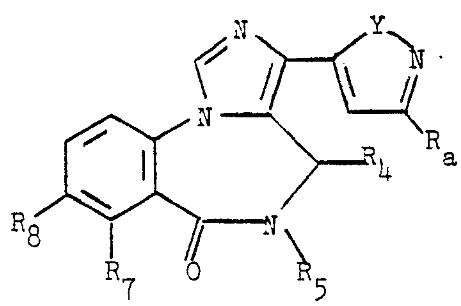

($I_B$)

dans laquelle Y, $R_a$, $R_4$, $R_5$, $R_7$ et $R_8$ ont la signification déjà indiquée, puis le cas échéant, salifie les produits de formule (I) ainsi obtenus.

Dans la formule générale (I) et dans ce qui suit :

- par radical alkyle linéaire ou ramifié renfermant de 1 à 5 atomes de carbone, on entend de préférence un radical méthyle, éthyle, propyle, isopropyle, butyle, isobutyle, terbutyle ou pentyle,
- par radical cycloalkyle renfermant de 3 à 6 atomes de carbone, on entend de préférence un radical cyclopropyle, cyclobutyle, cyclopentyle ou cyclohexyle,
- par radical alkényle linéaire ou ramifié renfermant de 2 à 5 atomes de carbone, on entend de préférence un radical vinyle ou propèn-2-yle,
- par groupement alkylène ou alkénylène contenant 2 à 5 atomes de carbone, on entend de préférence les groupements éthényl, 1-propylényl, propényl ou butényl,
- par groupement éliminable, on entend de préférence un atome de chlore, de brome ou un groupement p-toluènesulfonyl.

Dans des conditions préférentielles de mise en oeuvre de l'invention :

- la réaction du produit de formule (II) avec le produit de formule (III) est effectuée au sein d'un solvant organique anhydre tel que le benzène en présence d'une base telle que la triéthylamine,
- l'oxydation du produit de formule ($I_A$) est effectuée au moyen d'un oxydant doux tel que l'oxyde de manganèse.

Les produits de formule (I) présentent un caractère basique. On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les sels d'addition avec les acides minéraux ou organiques peuvent être, par exemple, les sels formés avec les acides chlorhydrique, bromhydrique, iodhydrique, nitrique, sulfurique, phosphorique, propionique,

formique, benzoïque, maléique, fumarique, succinique, tartrique, citrique, oxalique, glyoxylique, aspartique, alcanesulfoniques tels que l'acide méthanesulfonique et arylsulfoniques tel que l'acide benzènesulfonique.

L'invention a également pour objet à titre de produits industriels nouveaux les produits de formule ($I_C$) dont les noms suivent :

- la 3-(4,5-dihydro 3-cyclopropylisoxazol-5-yl) 5,6-dihydro 5-méthyl 6-oxo-4H-imidazo [1,5-a] [1,4]-benzodiazépine ;
- la 3-(4,5-dihydro 3-phénylisoxazol-5-yl) 5,6-dihydro 5-méthyl 6-oxo-4H-imidazo [1,5-a] [1,4]-benzodiazépine ;
- la 3-(4,5-dihydro 3-méthylisoxazol-5-yl) 5,6-dihydro 5-méthyl 6-oxo-4H-imidazo [1,5-a] [1,4]-benzodiazépine ;
- la 3-(4,5-dihydro 3-éthylisoxazol-5-yl) 5,6-dihydro 5-méthyl 6-oxo-4H-imidazo [1,5-a] [1,4]-benzodiazépine ;
- la 3-(3-cyclopropylisoxazol-5-yl) 5,6-dihydro 5-méthyl 6-oxo-4H-imidazo [1,5-a] [1,4]-benzodiazépine ;
- la 5,6-dihydro 5-méthyl 6-oxo 3-(3-phénylisoxazol-5-yl) 4H-imidazo [1,5-a] [1,4]-benzodiazépine ;
- la 5,6-dihydro 5-méthyl 6-oxo 3-(3-méthylisoxazol-5-yl) 4H-imidazo [1,5-a] [1,4]-benzodiazépine ; et
- la 5,6-dihydro 5-méthyl 6-oxo 3-(3-éthylisoxazol-5-yl) 4H-imidazo [1,5-a] [1,4]-benzodiazépine ;

ainsi que leurs sels d'addition avec les acides.

L'invention a également pour objet un procédé de préparation des composés ($I_D$) suivants :

- la 3-(3-cyclopropylisoxazol-5-yl) 5,6-dihydro 5-méthyl 6-oxo-4H-imidazo [1,5-a] [1,4]-benzodiazépine ;
- la 5,6-dihydro 5-méthyl 6-oxo 3-(3-phénylisoxazol-5-yl) 4H-imidazo [1,5-a] [1,4]-benzodiazépine ;
- la 5,6-dihydro 5-méthyl 6-oxo 3-(3-méthylisoxazol-5-yl) 4H-imidazo [1,5-a] [1,4]-benzodiazépine ; et
- la 5,6-dihydro 5-méthyl 6-oxo 3-(3-éthylisoxazol-5-yl) 4H-imidazo [1,5-a] [1,4]-benzodiazépine ;

caractérisé en ce que l'on fait réagir respectivement un produit de formule ($I_E$) :

- la 3-(4,5-dihydro 3-cyclopropylisoxazol-5-yl) 5,6-dihydro 5-méthyl 6-oxo-4H-imidazo [1,5-a] [1,4]-benzodiazépine ;
- la 3-(4,5-dihydro 3-phénylisoxazol-5-yl) 5,6-dihydro 5-méthyl 6-oxo-4H-imidazo [1,5-a] [1,4]-benzodiazépine ;
- la 3-(4,5-dihydro 3-méthylisoxazol-5-yl) 5,6-dihydro 5-méthyl 6-oxo-4H-imidazo [1,5-a] [1,4]-benzodiazépine ;
- la 3-(4,5-dihydro 3-éthylisoxazol-5-yl) 5,6-dihydro 5-méthyl 6-oxo-4H-imidazo [1,5-a] [1,4]-benzodiazépine ;

avec un oxydant doux tel que le dioxyde de manganèse.

Les nouveaux produits, objet de la présente invention, possèdent de très intéressantes propriétés pharmacologiques, on note en particulier de faibles propriétés agonistes inverses pour certains, et tous présentent une plus longue durée d'action en comparaison d'autres imidazobenzodiazépines.

Ces propriétés sont illustrées plus loin dans la partie expérimentale.

Ces propriétés justifient l'utilisation des nouvelles imidazobenzodiazépines de formule (I) ainsi que leurs sels pharmaceutiquement acceptables, à titre de médicaments.

La présente invention a aussi également pour objet l'application à titre de médicaments des nouvelles imidazobenzodiazépines telles que définies par les formules ($I_A$) et ($I_B$) ainsi que de leurs sels d'addition avec les acides pharmaceutiquement acceptables.

Parmi les médicaments préférés de l'invention, on retient tout particulièrement la 5,6-dihydro 5-méthyl 6-oxo 3-(3-méthylisoxazol-5-yl) 4H-imidazo [1,5-a] [1,4] benzodiazépine et ses sels d'addition avec les acides minéraux ou organiques pharmaceutiquement acceptables.

Ces médicaments trouvent, par exemple, leur emploi dans le traitement des troubles de la mémoire, notamment en gériatrie, des troubles de la sénescence cérébrale. Certains produits peuvent également être utilisés dans le traitement de l'obésité ainsi que comme tranquillisant mineur dans le traitement de certaines agitations ou irritabilité et dans certaines formes d'épilepsie.

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être

incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) peuvent être préparés comme indique dans la demande de brevet européen publiée sous le n° 285 837.

Il va être donné maintenant à titre non limitatif des exemples de mise en oeuvre de l'invention.

**Exemple 1 : 3-(4,5-dihydro 3-cyclopropylisoxazol-5-yl) 5,6-dihydro 5-méthyl 6-oxo 4H-imidazo [1,5-a] [1,4] benzodiazépine.**

On agite 0,5 g de 4,5-dihydro 5-méthyl 3-vinyl 6-oxo 4H-imidazo [1,5-a] [1,4] benzodiazépine dans 15 $cm^3$ de benzène sous atmosphère inerte, en présence de 0,3 g de cyclopropylchloro-oxime et ajoute en 30 minutes, 0,4 $cm^3$ de triéthylamine dans 1,5 $cm^3$ de benzène. On agite pendant 24 heures, ajoute un supplément de 0,5 g de cyclopropylchloro-oxime et 0,5 $cm^3$ de triéthylamine dans 2 $cm^3$ de benzène. On maintient le milieu réactionnel sous agitation pendant 60 heures, évapore le solvant, purifie le résidu par chromatographie (éluant : chlorure de méthylène-acétate d'éthyle) et obtient le produit attendu avec un rendement de 63%.

En opérant comme indiqué à l'exemple 1, on a préparé les composés suivants :

**Exemple 2 : la 3-(4,5-dihydro 3-phénylsiloxazol-5-yl) 5,6-dihydro 5-méthyl 6-oxo-4H-imidazo [1,5-a] [1,4]-benzodiazépine ;**

**Exemple 3 : la 3-(4,5-dihydro 3-méthylisoxazol-5-yl) 5,6-dihydro 5-méthyl 6-oxo-4H-imidazo [1,5-a] [1,4]-benzodiazépine ;**

**Exemple 4 : la 3-(4,5-dihydro 3-éthylisoxazol-5-yl) 5,6-dihydro 5-méthyl 6-oxo-4H-imidazo [1,5-a] [1,4]-benzodiazépine ;**

**Exemple 5 : la 3-(3-cyclopropylisoxazol-5-yl) 5,6-dihydro 5-méthyl 6-oxo-4H-imidazo [1,5-a] [1,4]-benzodiazépine.**

On chauffe au reflux 66 mg de 5-(4,5-dihydro 3-cyclopropylisoxazol-5-yl) 5,6-dihydro 5-méthyl 6-oxo-4H-imidazo [1,5-a] [1,4]-benzodiazépine dans 2 $cm^3$ de chloroforme en présence de 0,33 g de dioxyde de manganèse et ajoute toutes les heures un supplément de dioxyde de manganèse. Après 4 heures et demie, la réaction est terminée. On filtre sur Celite et purifie par chromatographie pour obtenir le produit attendu avec un rendement de 76%.

En opérant comme indiqué à l'exemple 5, on a préparé les composés suivants :

**Exemple 6 : la 5,6-dihydro 5-méthyl 6-oxo 3-(3-phénylisoxazol-5-yl) 4H-imidazo [1,5-a] [1,4]-benzodiazépine.**

**Exemple 7 : la 5,6-dihydro 5-méthyl 6-oxo 3-(3-méthylisoxazol-5-yl) 4H-imidazo [1,5-a] [1,4]-benzodiazépine.**

**Exemple 8 : la 5,6-dihydro 5-méthyl 6-oxo 3-(3-éthylisoxazol-5-yl) 4H-imidazo [1,5-a] [1,4]-benzodiazépine.**

TABLEAU I

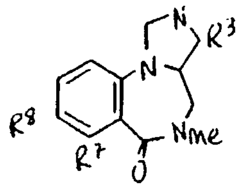

| Ex. | $R^3$ | $R^7$ | $R^8$ | Rendt | F°C | IR cm$^{-1}$ |
|---|---|---|---|---|---|---|
| 2 | 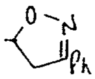 | H | H | 87% | 189-191°C | (KBr.) 2900,1625,1480,1380,1340,1220,880, 820,745,680 |
| 3 | 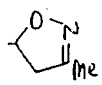 | H | H | 61%·-90% | 176-177°C | (KBr.) 3450(br),1625,1480,1420,1220,1035, 750,695 |
| 4 | 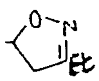 | H | H | 77%·-90% | 61-64°C | (KBr.) 3460,3420,1630,1485,1385,1330,1230 750 |
| 1 | 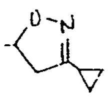 | H | H | 63% | 79-81°C | (KBr.)3400(br.),2980,2890,1620,1475,1370, 1320,1220,1030,740,690,625 |
| 5 | 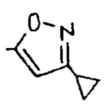 | H | H | 76% | 204-209°C | -max(KBr.)3010,1630,1595,1490,1485,1390, 1300,1230,1200,1030,925,750,695 |
| 6 | 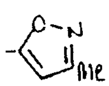 | H | H | 69% | 274-278°C | -max.3090,1630,1595,1495,1460,1420,1390, 1330,1310,1250,1230,1205,1150,1120,1040, 925,805,755,715,700 |
| 7 | 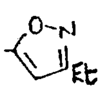 | H | H | 66% | 216-218°C | -max.3530,3090,1030,1490,1485,1450,1390, 1330,1310,1230,1200,1115,1035,925,810,760 730,695,645 |

TABLEAU I (suite)

| Ex. | RMN | Formule MWt | C Calc Trouvé | H | N |
|---|---|---|---|---|---|
| 2 | $(CDCl_3)$: 3.09(3H,s.);3.74-3.80(2H,ABq); 5.99(1H,+);7.49(4H,m.);7.68(4H,m.); 7.85(1H,d.);8.77(1H,s.) | $C_{21}H_{18}N_4O_2$ 358.40 | | | |
| 3 | $(CDCl_3)$; 2.07(3H,s.);3.24(3H,s.)3.37 (2H,br.d.);4.39-4.67(2H,ABq);5.76(1H,t.); 7.35(1H,dd);7.57(2H,m);7.84(1H,s);8.01(1H,dd) | $C_{16}H_{16}N_4O_2$ 296.33 | 64.85<br>64.73 | 5.44<br>5.54 | 18.91<br>18.57 |
| 4 | $(CDCl_3)$; 1M(3H,t);2.38(2H,q.);3.24(3H,s); 3.37(2H,ABq.);4.31-4.68(2H,br.m.);5.71(1H,t); 7.27(1H,dd.);7.39-7.65(2H,m);7.84(1H,s.); 8.02-8.06(1H,dd) | $C_{17}H_{18}N_4O_2$ 310.35 | | | |
| 1 | $(CDCl_3)$: 0.77-1.0(4H,m.);1.79-1.89(1H,m.); 3.20-3.24(5H,m.);4.31-4.76(2H,br.ABq);3.69-5.78(1H,t.);7.35-7.39(1H,dd.);7.44-7.64(2H,m.); 7.83(1H,s.);8.01-8.06(1H,dd). | $C_{18}H_{18}N_4O_2$ 322.37 | | | |
| 5 | 0.85-1.15(4H,m);2.00-2.17(1H,m.);3.24-3.29 (3H,s);4.66-5.03(2H,ABq.);6.35(1H,s.); 7.42-7.69(3H,m.);7.95(1H,s.);8.04-8.09(1Hdd.) | $C_{18}H_{16}N_4O_2$ 320.35 | | | |
| 6 | 2.38(3H,s.);3.28(3H,s.);4.48-5.05(2H,ABq.); 6.54(1H,s.);7.42-7.46(1H,dd.)7.50-7.70(2H,m.); 7.95(1H,s.);8.06-8.10(1Hdd.) | $C_{16}H_{14}N_4O_2$ 294.31 | 65.30 | 4.79 | 19.04 |
| 7 | 1.31-1.38(3H,t.);2.72-2.83(2H,q.);3.29(3H,s.); 4.55-4.98(2H,ABq.);6.57(1H,s.);7.42-7.46 (1H,dd.);7.49-7.70(2H,m.);7.95(1H,s.); 8.06-8.10(1H,dd). | $C_{17}H_{16}N_4O_2$ 308.339 | 66.22 | 5.23 | 18.17 |

**Exemple 9 :**

On a préparé des comprimés répondant à la formulation suivante :

| | |
|---|---|
| - Composé de l'exemple 7 ..... | 20 mg |
| - Excipient q.s.p. un comprimé terminé à ..... | 150 mg |
| (Composition de l'excipient : lactose, amidon, talc, stéarate de magnésium). | |

## ETUDE PHARMACOLOGIQUE

Les composés sont des agents qui agissent sur les récepteurs des benzodiazépines du cerveau ; certains peuvent être utiles dans le traitement de certaines formes d'épilepsie, certains peuvent être utiles comme tranquilisants légers et certains comme hypnotiques.

L'étude en screening sur la liaison aux récepteurs des benzodiazépines (F.R.B.) a été effectuée selon la méthode décrite dans le brevet anglais GB. 2 128 989.

La mesure in vivo de la liaison aux récepteurs des benzodiazépines a été effectuée selon la méthode décrite par Goeders N.E. et Kuhar M.J. Life, Sciences (1985) 37 , 345.

TABLEAU II

| Composé | FRB en nM | liaison in vivo DE 50 mg/kg IP |
|---|---|---|
| 5 | 87 | 2,0 |
| 7 | 40 | 0,62 |
| 8 | 118 | > 3,0 |

## Revendications

1.- Nouveau procédé de préparation des produits de formule générale (I) :

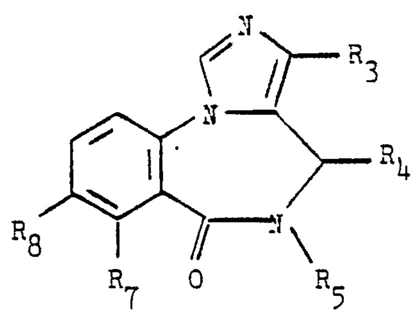

(I)

dans laquelle $R_3$ représente un groupement

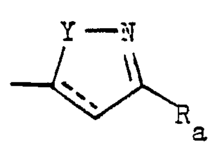

dans lequel $R_a$ représente un atome d'hydrogène, un groupement alkyle, linéaire ou ramifié renfermant de 1 à 5 atomes de carbone, un groupement cycloalkyle renfermant de 3 à 6 atomes de carbone, un groupement phényl, Y représente un atome d'oxygène ou de soufre, le trait pointillé représente une seconde liaison éventuelle,

$R_4$ et $R_5$, identiques ou différents, représentent un atome d'hydrogène, un radical alkyle linéaire ou ramifié renfermant de 1 à 5 atomes de carbone, un radical alkényl linéaire ou ramifié renfermant de 2 à 5 atomes de carbone, ou $R_4$ et $R_5$ forment ensemble un groupement alkylène ou alkénylène renfermant de 2 à 5 atomes de carbone,

$R_7$ et $R_8$, identiques ou différents, représentent un atome d'hydrogène, un atome d'halogène ou un groupement $CX_3$ dans lequel X représente un atome d'halogène ou encore un groupement nitro ou nitrile

ainsi que de leurs sels d'addition avec les acides minéraux ou organiques, procédé de préparation caractérisé en ce que l'on fait réagir un produit de formule (II) :

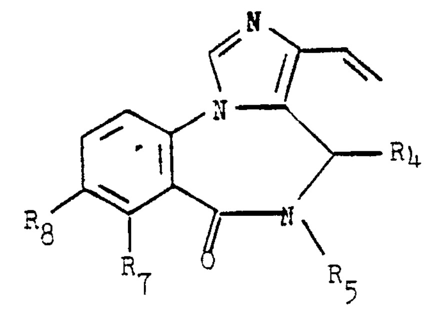

(II)

dans laquelle $R_4$, $R_5$, $R_7$ et $R_8$ ont la signification déjà indiquée, avec un produit de formule (III) :

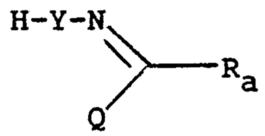

(III)

dans laquelle Y et $R_a$ ont la signification déjà indiquée et Q représente un groupement éliminable, pour obtenir un produit de formule ($I_A$) :

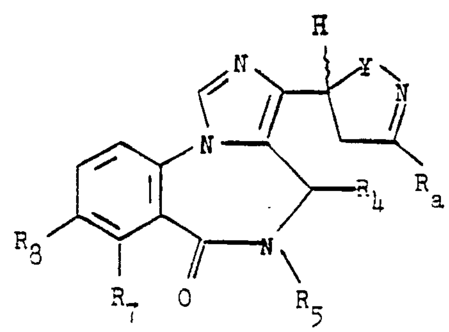

($I_A$)

dans laquelle Y, $R_a$, $R_4$, $R_5$, $R_7$ et $R_8$ ont la signification déjà indiquée, puis le cas échéant, fait réagir ce dernier avec un oxydant doux pour obtenir un produit de formule ($I_B$) :

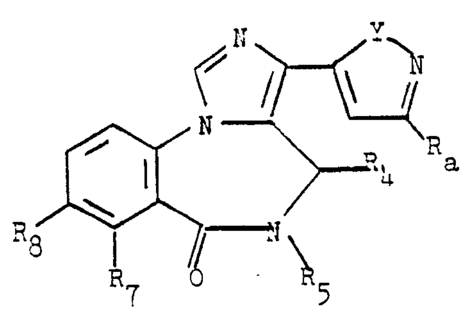

($I_B$)

dans laquelle Y, $R_a$, $R_4$, $R_5$, $R_7$ et $R_8$ ont la signification déjà indiquée, puis le cas échéant, salifie les produits de formule (I) ainsi obtenus.

2.- Procédé de préparation selon la revendication 1, caractérisé en ce que :

- la réaction du produit de formule (II) avec le produit de formule (III) est effectuée au sein d'un solvant organique anhydre tel que le benzène en présence d'une base telle que la triéthylamine,
- l'oxydation du produit de formule ($I_A$) est effectuée au moyen d'un oxydant doux tel que l'oxyde de manganèse.

3.- A titre de produits industriels nouveaux, les produits de formule ($I_A$) dont les noms suivent :
- la 3-(4,5-dihydro 3-cyclopropylisoxazol-5-yl) 5,6-dihydro 5-méthyl 6-oxo-4H-imidazo [1,5-a] [1,4]-benzodiazépine ;
- la 3-(4,5-dihydro 3-phénylisoxazol-5-yl) 5,6-dihydro 5-methyl 6-oxo-4H-imidazo [1,5-a] [1,4]-benzodiazépine ;
- la 3-(4,5-dihydro 3-méthylisoxazol-5-yl) 5,6-dihydro 5-méthyl 6-oxo-4H-imidazo [1,5-a] [1,4]-benzodiazépine ;
- la 3-(4,5-dihydro 3-éthylisoxazol-5-yl) 5,6-dihydro 5-méthyl 6-oxo-4H-imidazo [1,5-a] [1,4]-benzodiazépine ;
et leurs sels d'addition avec les acides minéraux ou organiques.

4.- A titre de produits industriels nouveaux, les produits de formule ($I_B$) dont les noms suivent :
- la 3-(3-cyclopropylisoxazol-5-yl) 5,6-dihydro 5-méthyl 6-oxo-4H-imidazo [1,5-a] [1,4]-benzodiazépine ;
- la 5,6-dihydro 5-methyl 6-oxo 3-(3-phénylisoxazol-5-yl) 4H-imidazo [1,5-a] [1,4]-benzodiazépine ;
- la 5,6-dihydro 5-méthyl 6-oxo 3-(3-méthylisoxazol-5-yl) 4H-imidazo [1,5-a] [1,4]-benzodiazépine ;
- la 5,6-dihydro 5-méthyl 6-oxo 3-(3-éthylisoxazol-5-yl) 4H-imidazo [1,5-a] [1,4]-benzodiazépine ;
ainsi que leurs sels d'addition avec les acides minéraux ou organiques.

5.- Médicaments, caractérisés en ce qu'ils sont constitués par les nouvelles imidazobenzodiazépines répondant à la formule ($I_A$) de la revendication 3, ainsi que par leurs sels pharmaceutiquement acceptables.

6.- Médicaments, caractérisés en ce qu'ils sont constitués par les nouvelles imidazobenzodiazépines répondant à la formule ($I_B$) de la revendication 4, ainsi que par leurs sels pharmaceutiquement acceptables.

7.- Médicaments selon la revendication 6, caractérisés en ce qu'ils sont constitués par la 5,6-dihydro 5-méthyl 6-oxo 3-(3-méthylisoxazol-5-yl) 4H-imidazo [1,5-a] [1,4] benzodiazépine et ses sels avec les acides minéraux ou organiques pharmaceutiquement acceptables.

8.- Compositions pharmaceutiques, caractérisées en ce qu'elles renferment à titre de principe actif, l'un au moins des médicaments tels que définis à l'une quelconque des revendications 5 à 7.

Revendications pour l'Etat contractant suivant: ES

1.- Nouveau procédé de préparation des produits de formule générale (I) :

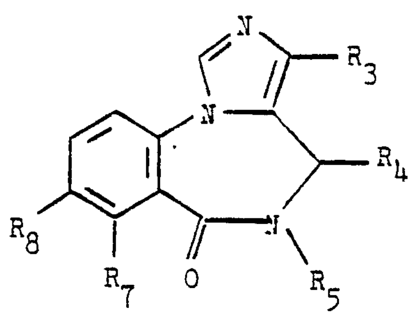

(I)

dans laquelle $R_3$ représente un groupement

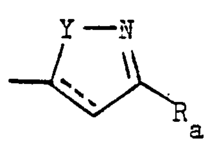

dans lequel $R_a$ représente un atome d'hydrogène, un groupement alkyle, linéaire ou ramifié renfermant de 1 à 5 atomes de carbone, un groupement cycloalkyle renfermant de 3 à 6 atomes de carbone, un groupement phényl, Y représente un atome d'oxygène ou de soufre, le trait pointillé représente une seconde liaison éventuelle,
$R_4$ et $R_5$, identiques ou différents, représentent un atome d'hydrogène, un radical alkyle linéaire ou ramifié renfermant de 1 à 5 atomes de carbone, un radical alkényl linéaire ou ramifié renfermant de 2 à 5 atomes de carbone, ou $R_4$ et $R_5$ forment ensemble un groupement alkylène ou alkénylène renfermant de 2 à 5 atomes de carbone,
$R_7$ et $R_8$, identiques ou différents, représentent un atome d'hydrogène, un atome d'halogène ou un groupement $CX_3$ dans lequel X représente un atome d'halogène ou encore un groupement nitro ou nitrile

ainsi que de leurs sels d'addition avec les acides minéraux ou organiques, procédé de préparation caractérisé en ce que l'on fait réagir un produit de formule (II) :

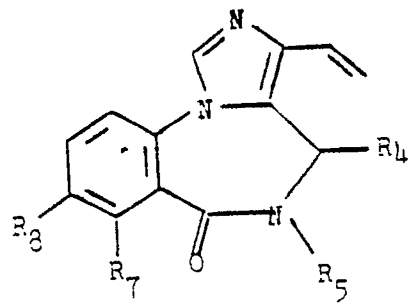

(II)

dans laquelle $R_4$, $R_5$, $R_7$ et $R_8$ ont la signification déjà indiquée, avec un produit de formule (III) :

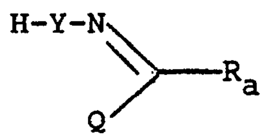

(III)

dans laquelle Y et $R_a$ ont la signification déjà indiquée et Q représente un groupement éliminable, pour obtenir un produit de formule ($I_A$) :

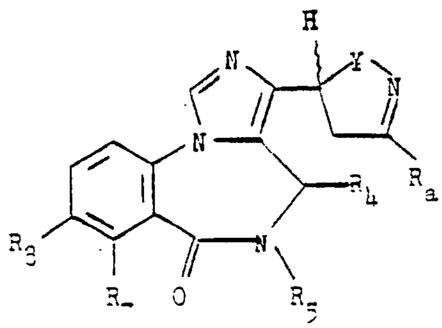

($I_A$)

dans laquelle Y, $R_a$, $R_4$, $R_5$, $R_7$ et $R_8$ ont la signification déjà indiquée, puis le cas échéant, fait réagir ce dernier avec un oxydant doux pour obtenir un produit de formule ($I_B$) :

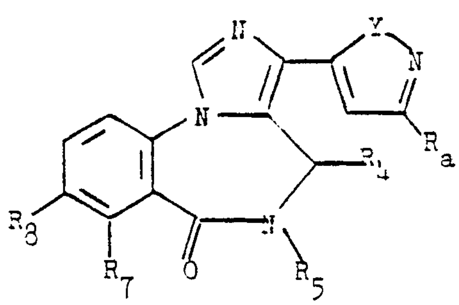

($I_B$)

dans laquelle Y, $R_a$, $R_4$, $R_5$, $R_7$ et $R_8$ ont la signification déjà indiquée, puis le cas échéant, salifie les produits de formule (I) ainsi obtenus.

2.- Procédé de préparation selon la revendication 1, caractérisé en ce que :

- la réaction du produit de formule (II) avec le produit de formule (III) est effectuée au sein d'un solvant organique anhydre tel que le benzène en présence d'une base telle que la triéthylamine,
- l'oxydation du produit de formule ($I_A$) est effectuée au moyen d'un oxydant doux tel que l'oxyde de manganèse.

Revendications pour l'Etat contractant suivant: GR

1.- Nouveau procédé de préparation des produits de formule générale (I) :

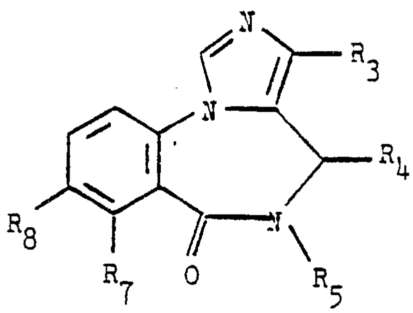

(I)

dans laquelle $R_3$ représente un groupement

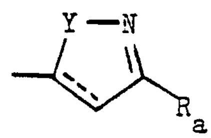

dans lequel $R_a$ représente un atome d'hydrogène, un groupement alkyle, linéaire ou ramifié renfermant de 1 à 5 atomes de carbone, un groupement cycloalkyle renfermant de 3 à 6 atomes de carbone, un groupement phényl, Y représente un atome d'oxygène ou de soufre, le trait pointillé représente une seconde liaison éventuelle,
$R_4$ et $R_5$, identiques ou différents, représentent un atome d'hydrogène, un radical alkyle linéaire ou ramifié renfermant de 1 à 5 atomes de carbone, un radical alkényl linéaire ou ramifié renfermant de 2 à 5 atomes de carbone, ou $R_4$ et $R_5$ forment ensemble un groupement alkylène ou alkénylène renfermant de 2 à 5 atomes de carbone,
$R_7$ et $R_8$, identiques ou différents, représentent un atome d'hydrogène, un atome d'halogène ou un groupement $CX_3$ dans lequel X représente un atome d'halogène ou encore un groupement nitro ou nitrile ainsi que de leurs sels d'addition avec les acides minéraux ou organiques,
procédé de préparation caractérisé en ce que l'on fait réagir un produit de formule (II) :

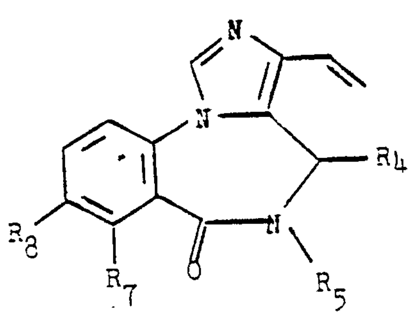

(II)

dans laquelle $R_4$, $R_5$, $R_7$ et $R_8$ ont la signification déjà indiquée, avec un produit de formule (III) :

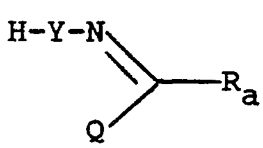

(III)

dans laquelle Y et $R_a$ ont la signification déjà indiquée et Q représente un groupement éliminable, pour obtenir un produit de formule ($I_A$) :

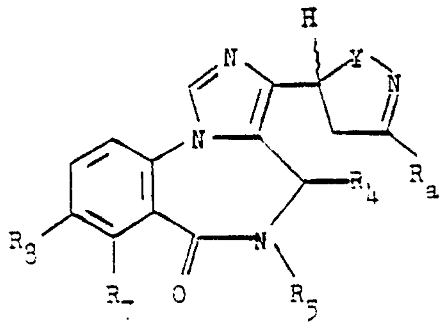

$(I_A)$

dans laquelle Y, $R_a$, $R_4$, $R_5$, $R_7$ et $R_8$ ont la signification déjà indiquée, puis le cas échéant, fait réagir ce dernier avec un oxydant doux pour obtenir un produit de formule ($I_B$) :

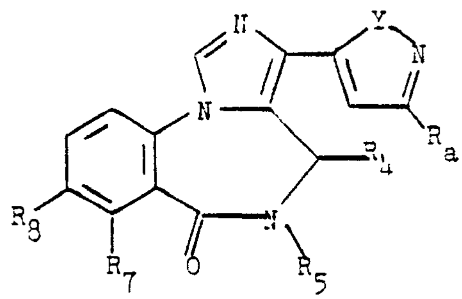

$(I_B)$

dans laquelle Y, $R_a$, $R_4$, $R_5$, $R_7$ et $R_8$ ont la signification déjà indiquée, puis le cas échéant, salifie les produits de formule (I) ainsi obtenus.

2.- Procédé de préparation selon la revendication 1, caractérisé en ce que :

- la réaction du produit de formule (II) avec le produit de formule (III) est effectuée au sein d'un solvant organique anhydre tel que le benzène en présence d'une base telle que la triéthylamine,
- l'oxydation du produit de formule ($I_A$) est effectuée au moyen d'un oxydant doux tel que l'oxyde de manganèse.

3.- Procédé selon la revendication 1, caractérisé en ce que l'on utilise au départ des composés de formule (II) et (III) choisis de manière telle que l'on prépare l'un quelconque des produits dont les noms suivent :

- la 3-(4,5-dihydro 3-cyclopropylisoxazol-5-yl) 5,6-dihydro 5-méthyl 6-oxo-4H-imidazo [1,5-a] [1,4]-benzodiazépine ;
- la 3-(4,5-dihydro 3-phénylisoxazol-5-yl) 5,6-dihydro 5-méthyl 6-oxo-4H-imidazo [1,5-a] [1,4]-benzodiazépine ;
- la 3-(4,5-dihydro 3-méthylisoxazol-5-yl) 5,6-dihydro 5-méthyl 6-oxo-4H-imidazo [1,5-a] [1,4]-benzodiazépine ;
- la 3-(4,5-dihydro 3-éthylisoxazol-5-yl) 5,6-dihydro 5-méthyl 6-oxo-4H-imidazo [1,5-a] [1,4]-benzodiazépine ;

et leurs sels d'addition avec les acides minéraux ou organiques.

4.- Procédé selon la revendication 1, caractérisé en ce que l'on soumet l'un quelconque des produits selon la revendication 3 à l'action d'un lxydant doux pour obtenir respectivement l'un des produits dont les noms suivent :

- la 3-(3-cyclopropylisoxazol-5-yl) 5,6-dihydro 5-méthyl 6-oxo-4H-imidazo [1,5-a] [1,4]-benzodiazépine ;
- la 5,6-dihydro 5-méthyl 6-oxo 3-(3-phénylisoxazol-5-yl) 4H-imidazo [1,5-a] [1,4]-benzodiazépine ;
- la 5,6-dihydro 5-méthyl 6-oxo 3-(3-méthylisoxazol-5-yl) 4H-imidazo [1,5-a] [1,4]-benzodiazépine ;
- la 5,6-dihydro 5-méthyl 6-oxo 3-(3-éthylisoxazol-5-yl) 4H-imidazo [1,5-a] [1,4]-benzodiazépine ;

ainsi que leurs sels d'addition avec les acides minéraux ou organiques.

5.- Procédé de préparation des compositions pharmaceutiques caractérisé en ce que l'on met à titre de principe actif l'un au moins des produits de formule (I) telle que définie à la revendication 1 ou l'un au moins de leurs sels pharmaceutiquement acceptables sous une forme destinée à cet usage.